# EUROPEAN PATENT APPLICATION

(11) **EP 4 140 503 A1**
(43) Date of publication of application: **01.03.2023**
(21) Application number: 20925200.6
(22) Date of filing: 30.12.2020
(51) Int. Cl.: A61K 45/00, A61P 3/10

(54) **USE OF EPHB4 AS TARGET IN SCREENING OF DRUGS OR MODELS FOR INCREASING INSULIN SENSITIVITY**

(30) Priority: 17.03.2020 CN 202010188663
(71) Applicant: INSTITUTE OF MATERIA MEDICA, CHINESE ACADEMY OF MEDICAL SCIENCES, Beijing 100050 (CN)
(72) Inventor: LI, Pingping, Beijing 100050 (CN); LIU, Xingfeng, Beijing 100050 (CN); CUI, Bing, Beijing 100050 (CN); WANG, Kai, Beijing 100050 (CN); CHEN, Jingwen, Beijing 100050 (CN); HOU, Shaocong, Beijing 100050 (CN); KONG, Lijuan, Beijing 100050 (CN); JIANG, Qian, Beijing 100050 (CN); MA, Chunxiao, Beijing 100050 (CN)
(74) Representative: Deb, Roona
(86) International application number: PCT/CN2020/141441
(87) International publication number: WO 2021/184918

(57) **Abstract**

The present invention belongs to the technical field of protein and genetic engineering, and specifically discloses use of erythropoietin-producing hepatocyte receptor B4 as a target in screening and preparing a biological formulation or medicament for increasing insulin sensitivity to insulin. Also the use of erythropoietin-producing hepatocyte receptor B4 in preparing an insulin-sensitized mouse model is disclosed. On the basis of insulin signal regulation, a protein EphB4 capable of interacting with insulin receptor (InsR) is found. The protein can interact with InsR, and insulin stimulation can promote the interaction between the two, which provides a certain basis for insulin resistance in the case of hyperinsulinemia. Over-expression of EphB4 can promote the degradation of InsR, while inhibition of EphB4 can enhance insulin sensitivity to insulin and improve insulin resistance.

## Description

### Technical field

The invention relates to the technical field of protein and genetic engineering, in particular to use of EphB4, a new target for the prevention and treatment of insulin resistance and related diseases.

### Background technology

In China, with the changes of dietary habits and lifestyle, the incidence of diabetes is rising rapidly. By 2010, the morbidity rate of diabetes among Chinese adults had reached 11.6 percent, and the proportion of pre-diabetes was as high as 50.1 percent. By 2015, the number of Chinese adults with diabetes reached 109.6 million, making China the country with the largest diabetic population in the world. At present, diabetes has become a widely concerned public health issue. Without timely intervention and treatment, type 2 diabetes may induce cardiovascular disease, kidney disease, eye disease and other diabetes-related metabolic syndrome, which seriously affects the health of patients and even endangers their lives.

Insulin secretion and its signal regulation play an important role in glucose metabolism and homeostasis. Insulin resistance is one of the important pathological features of type 2 diabetes mellitus. Many studies have shown that insulin resistance, as important pathogenesis of type 2 diabetes, is caused by a variety of factors such as chronic tissue inflammation, blocked insulin signal transduction and disturbance of intestinal flora. In addition, in the case of insulin resistance, there will be an increase in insulin compensation, leading to the occurrence of hyperinsulinemia.

At present, among the drugs used in clinical treatment of diabetes, thiazolidinedione (TZD) compounds are the only insulin sensitizer that can clearly improve insulin resistance. Its target of action is PPARγ, which is a superfamily of nuclear receptors in mammals. But TZD drugs have been withdrawn from the market or restricted due to side effects such as weight gain and cardiovascular risk. Therefore, there is no good insulin sensitizer in the current application of drugs. In view of the above problems, it is urgent to find a new insulin sensitizer to solve the existing problems.

### Contents of the invention

The technical problem solved by the invention is to provide a use in screening and preparing biological agents or drugs that increase insulin sensitivity by targeting erythropoietin-producing hepatocyte receptor B4.

Another technical problem solved by the invention is to provide a use of erythropoietin-producing hepatocyte receptor B4 in the preparation of insulin-sensitized mouse model. It was specifically verified in animal level that the ability of glucose tolerance and clearance could be improved in mouse models by downregulating the expression of EphB4 in mice.

To solve the above technical problems, the invention provides the following scheme:
The first aspect of the technical scheme of the invention is to provide a use of erythropoietin-producing hepatocyte receptor B4 in screening or preparation of a biological agent or a drug for increasing insulin sensitivity, wherein the erythropoietin-producing hepatocyte receptor B4 is used as a target.

Preferably, said biological agent or drug is used for preventing, alleviating or treating insulin resistance or a disease related to insulin resistance.

Preferably, said insulin resistance or disease related to insulin resistance is diabetes mellitus, hyperinsulinemia, lipid metabolism disorder, obesity or glucose intolerance.

Preferably, said biological agent or drug is used for inhibiting the interaction of EphB4 with insulin receptors, or increasing protein level of insulin receptors or level of phosphorylated Akt, or enhancing ability of glucose tolerance and clearance.

The corresponding amino acid sequence of said insulin receptor is shown in NP_001073285.1, and was constructed in pCMV3 vector and was fused to express flag label. The corresponding amino acid sequence of EPHB4 is shown in NP_004435.3, and was constructed in pCMV5 vector and was fused with HA label. The interaction between insulin receptor and EPHB4 was demonstrated in both directions under the condition of their overexpression.

In this study, the inventors discovered the interaction between endogenous insulin receptor and EPHB4 in HepG2 cells; the interaction between endogenous insulin receptor and EPHB4 was also demonstrated in hepatocytes differentiated from human pluripotent hepatocytes. The inventors also found that insulin stimulation can promote the interaction between insulin receptor and EPHB4 in HepG2 cells.

The inventors provided evidence that EphB4 promotes degradation of insulin receptor proteins by lysosomal pathway, and inhibition of EphB4 increases the level of phosphorylated Akt. Specifically, overexpression of EPHB4 by adenovirus vectors in primary hepatocytes reduced the level of phosphorylated Akt and the level of insulin receptor proteins. In addition, inhibition of EphB4 by treating primary mouse liver cells with secondary cholic acid which is a drug that inhibits EphB4 (LCA, also known as 3α-hydroxy-5β-cholanoic acid, 3α-hydroxy-5β-cholestane-24-acid, 5β-cholestane-24-acid-3α-alcohol, molecular formula C₂₄H₄₀O₃) increased the level of phosphorylated Akt. Treatment with proteasome pathway inhibitors and lysosomal pathway inhibitors, as well as detection of interactions with Rab7, provided evidence that EphB4 promotes insulin receptor degradation through the lysosomal pathway.

The second aspect of the technical scheme of the invention is to provide a use of erythropoietin-producing hepatocyte receptor B4 in preparing an insulin-sensitized mouse model.

Preferably, CRISPR-Cas9 technologyis used to construct a transgenic mice in which *EphB4* gene was knocked out tissue-specifically and two flanks of first exon were inserted with LoxP site, thereby an insulin-sensitized mouse model with *EphB4* tissue-specifically knockout is obtained.

Preferably, said insulin-sensitized mouse model with *EphB4* tissue-specifically knockout has enhanced ability of glucose tolerance and clearance.

### Beneficial technical effects

The invention discloses a use in screening and preparing biological agents or drugs that increase insulin sensitivity by targeting erythropoietin-producing hepatocyte receptor B4 (EphB4). Specifically, from the perspective of insulin signal regulation, an insulin receptor interaction protein EphB4 that can be used as an insulin sensitizer target was provided and EphB4 was found to interact with insulin receptors for the first time. The invention also proves that the interaction can be promoted by insulin stimulation at cellular level. EphB4 promotes degradation of insulin receptor protein through the lysosomal pathway, proving that inhibition of EphB4 can increase the level of phosphorylated Akt, which provides a certain basis for insulin resistance in the case of hyperinsulinemia. At the same time, it was also proved at the animal level that knockdown of EphB4 could improve ability of glucose tolerance and clearance of db/db mice, suggesting that inhibition of EphB4 could enhance insulin sensitivity and improve insulin resistance. The invention discovers a new target that can be used for prevention and treatment of insulin resistance and related diseases, and provides a new idea and target for the treatment of diabetes and for antidiabetic drug screening. Additionally, it is of great significance to solve the defects of existing drugs currently used in the treatment of diabetes.

### Figure Legends

In order to describe the technical scheme in embodiments of the invention or in prior art, the following is a brief introduction of the figures required for use in embodiments. It is obvious that the figures described below are only some embodiments of the invention, from which other figures can be obtained without creative effort by ordinary technicians in the field.
Fig. 1 shows the result of the interaction between insulin receptor and EphB4 obtained by immunoprecipitation experiment in this patent.
Fig. 2 shows EphB4 promotes the degradation of insulin receptors in this patent.
Fig. 3 shows overexpression of EphB4 by adenovirus vector inhibits insulin signaling in mice in this patent.
Fig. 4 shows down-regulation of EphB4 expression by lentiviral vector in db/db mice improves insulin resistance in this patent.
Fig. 5 shows liver specific knockout of EphB4 improves insulin sensitivity in mice fed with high fat diet in this patent.

### Specific implementation mode

Various exemplary embodiments of the invention are detailed below. The specification should not be considered as a restriction of the invention, but should be understood as a more detailed description of certain aspects, features and implementation schemes of the invention.

It should be understood that the terms described in the present invention are intended only to describe particular embodiments and are not intended to limit the present invention. In addition, the numerical range in the present invention shall be understood to specify each intermediate value between the upper and lower limits of the range. Each smaller range between any stated value or intermediate value within the stated range and any other stated value or intermediate value within the stated range is also included in the present invention. The upper and lower limits of these smaller ranges can be included or excluded independently.

Unless otherwise stated, all technical and scientific terms used herein have the same meaning generally understood by conventional technicians in the fields described in the present invention. Although the present invention only describes the preferred method and material, any method and material similar or equivalent to those described herein can also be used in the implementation or testing of the present invention. All literature mentioned in this manual is incorporated by reference to disclose and describe methods and/or materials related to the literature. In case of conflict with any incorporated literature, the contents of this manual shall prevail.

Without deviating from the scope or spirit of the invention, a variety of improvements and changes can be made to the specific implementation of the specification of the invention, which is obvious to the technician in the field. Other embodiments obtained from the specification of the present invention are obvious to the technician. This application manual and embodiments are only exemplary.

With regard to the words "including", "having", "containing", "involving" and so on, they are all open terms, that is, they mean to include but not to limit.

### Embodiment 1

### 1. Research methods and technical route

The present invention combines cell model and animal model. HEK293T cell, HepG2 cell, primary hepatocyte, C57BL/6J mice, *db*/*db* mice and insulin-sensitized animal model of *EphB4* liver-specific knockout mice (*EphB4* LKO) were used as research objects, and the corresponding cell or tissue samples were collected for the corresponding study.

Real-time quantitative PCR, western blotting, glucose tolerance test and insulin tolerance test in mice were used as the main methods.

### 2. Experimental material

The transfection agent Lipo3000 was purchased from Invitrogene Corporation. InsR, EphB4, Akt, and pAkt antibodies were purchased from Cell Signaling Technology, the article numbers were #3025 #14960 #4685 and #4060. Western blotting was used in a ratio of 1:1000 to 2000 and protein immunoprecipitation was used in a ratio of 1:100.

Adenovirus that overexpressing EphB4 and lentivirus with knocked down *EphB4* expression as well as corresponding control viruses were constructed and packaged by Shanghai Genechem Corporation. Correlation sequences: *EphB4* shRNA sequences 5'-GTTATGATCCTCACGGAAT-3', control shRNA sequences 5'-TTCTCCGAACGTGTCACGT-3' .

The inhibitors MG132(S2619), chloroquine (S4157) and LCA(S4003) were purchased from Selleck chemicals. Ammonium chloride (A9434) was purchased from Sigma Company.

During the glucose tolerance and insulin tolerance tests, the blood glucose of mice was measured by Roche superior gold extraction type glucose meter and corresponding blood glucose test paper.

The primer sequences that were used in real-time quantitative PCR are as follows:
EphB4-F: TATGCCACGATACGCTTCACC;
EphB4-R: AGCTTCGCTCTCGTAATAGAAGA;
36B4-F: AGATTCGGGATATGCTGTTGGC;
36B4-R: TCGGGTCCTAGACCAGTGTTC.

The relevant sequences of tissue-specifically knockout *EphB4* transgenic mice constructed by CRISPR-Cas9 are as follows:
The target sequences of CRISPR-Cas9 are 5'-GCCCGAGATCTTTACTCCCCGGG-3' and 5'-TTCTGGGCTGATCAAAGTGTGGG-3', corresponding sequences of sgRNA are 5'-CTATTTCTAGCTCTAAAACGGGGAGTAAAGATCTCGGGCCTATAGTGAGTC GTATTA-3' and 5'-CTATTTCTAGCTCTAAAACACACTTTGATCAGCCCAGCCTATAGTGAGTCGT ATT-3'.

PCR primer pairs for genotype identification are as follows: front 5'-AGTTCCTCCAAGTCCCCTAACACC-3', 5'-CGCGCACTGGTGAAAATCCC-3', wild type size is 287bp, mutant type size is 372bp; reverse 5'-GGAGAATCTGGGGAGTGGGACA-3', 5'-ACTCTCCTTTTTTGCTGGGCAAAAT-3', wild type size is 318bp, mutant type size is 410bp.

Statistical analysis: Values were expressed as mean and positive and negative standard deviations, and differences between the two groups were determined by student-Newman-Keuls test.

### 3. Experimental methods and results

### 3.1 Co-immunoprecipitation

HEK293T cells and human liver cancer cells HepG2 cells were cultured in DMEM medium containing 10% fetal bovine serum and streptomycin and penicillin (gibco,15140-122) in a constant temperature incubator of 37 °C and 5% CO₂. In the co-immunoprecipitation experiment, for overexpression conditions, the expression plasmids pCMV5-EphB4-HA and pCMV3-flag-InsR were transferred into the cells via transfection reagent Lipo3000 at 70%-80% cell confluence, and then the cells were collected 30-36 hours after transfection. As for endogenous protein, directly cleaved the cultured cells and stimulated 30min with insulin of 10nM before collecting the cells. After collecting the lysed cells, the corresponding antibodies of the target protein were added in accordance with the recommended proportion of the used antibodies for immunoprecipitation, and were rotated for 5-12 h in a 4 °C refrigerator. After rinsing, the protein-protein interaction was detected by Western blotting.

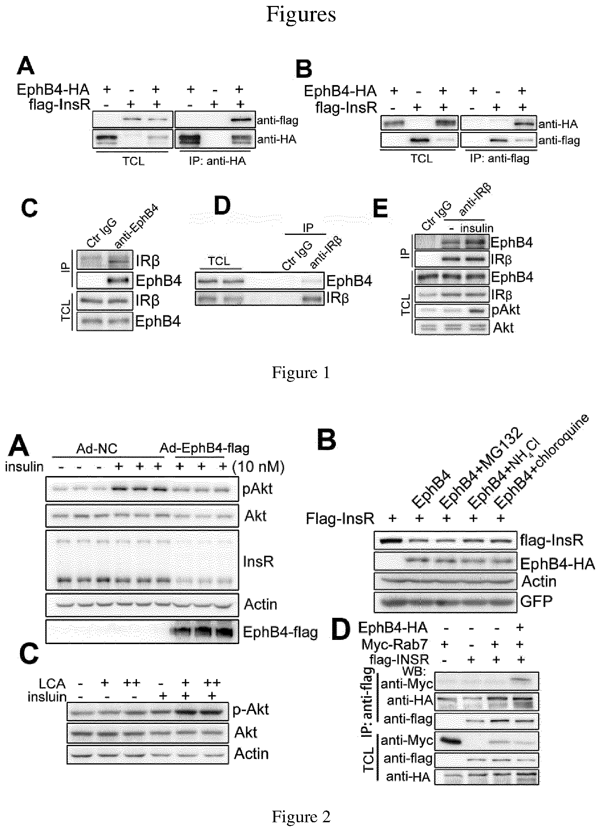

The results showed that insulin receptor with flag tag (flag-InsR) and EphB4 with HA tag (EphB4-HA) were overexpressed in HEK293T cells, and the interaction between insulin receptor and EphB4 could be detected by co-immunoprecipitation assay (as shown in Fig. A and B of Fig. 1).

The endogenous interaction between EphB4 and insulin receptors was also detected in HepG2 cells and hepatocytes induced by human pluripotent hepatocytes (as shown in Fig. C and D of Fig. 1). Moreover, HepG2 was treated with insulin (insulin10 nM) for half an hour, and then the interaction between them was detected by immunoprecipitation. The results showed that insulin stimulation could promote the interaction between insulin receptor and EphB4.

The above results show that there is an interaction between EphB4 and insulin receptor under both overexpression and endogenous conditions, and insulin stimulation can enhance the interaction between them.

### 3.2 EphB4 inhibitors increase the level of phosphorylated Akt

The mice were anesthetized by intraperitoneal injection of anesthetic, then abdominal cavity was opened, injected with type IV collagenase (Sigma, C5138) through portal vein, and fluid flew out of the inferior vena cava. After the liver tissue was fully digested, it was suspended with DMEM medium, flew through a cell sieve with a diameter of 70 µm, and was centrifugated, thereby the primary hepatocytes were obtained. The cells were resuspended in complete culture medium and dispersed into a petri dish or plate. After the cells were attached, they were treated with LCA (10 µM and 20 µM) overnight. Cells were stimulated with 10 nM insulin for 20 min before collection, and then the level of phosphorylated Akt was detected by western blotting.

The results showed that the treatment of primary mouse hepatocytes with Eph signal inhibitor lithocholic acid LCA could increase the level of phosphorylated Akt (as shown in Fig. 2C).

### 3.3 Overexpression of EphB4 reduces the level of phosphorylated Akt and insulin receptor

The mice were anesthetized by intraperitoneal injection of anesthetic, then abdominal cavity was opened, injected with type IV collagenase (Sigma, C5138) through portal vein, and fluid flew out of the inferior vena cava. After the liver tissue was fully digested, it was suspended with DMEM medium, flew through a cell sieve with a diameter of 70 µm, then the primary hepatocytes were obtained by centrifuging with 50×g centrifugal force. After washing twice with DMEM medium, the cells were re-suspended with complete culture medium and dispersed to petri dishes or culture plates. After the cells were adhered to the wall, the primary hepatocytes were infected with adenovirus containing EphB4, and EphB4 was overexpressed. When cells were infected with adenovirus, MOI=100. The infected cells were cultured for 16 h and collected for western blot detection.

The results showed that overexpression of EphB4 could reduce the level of phosphorylated Akt and the level of insulin receptor protein in primary hepatocytes (as shown in Fig. 2A).

### 3.4 EphB4 promotes the degradation of insulin receptor through lysosomal pathway

Insulin receptor (flag tagged insulin receptor) and EphB4 (HA tagged EphB4) were co-expressed in HEK293T cells. GFP was co-transfected as a reference for foreign proteins. The cells were treated with proteasome degradation pathway inhibitor MG132 (10 µM), lysosome degradation pathway inhibitor ammonium chloride (5 mM) and chloroquine (10 µM) for 8 h. Cells were collected to detect protein level of flag-InsR by Western blotting (as shown in Fig. 2B). Lysosomal degradation pathway inhibitors ammonium chloride and chloroquine could partially rescue the promoting effect of EphB4 on insulin receptor degradation to some extent, indicating that EphB4 promotes insulin receptor degradation through lysosomal pathway. At the same time, by comparing the interaction between InsR and late endocytosis body marker protein Rab7 with or without co-expression of EphB4, it can be seen that co-expression of EphB4 can promote the interaction between InsR and Rab7, indicating that EphB4 promotes InsR to enter late endocytosis corpuscles, and then enter the lysosome for degradation (Fig. 2D).

The results showed that EphB4 promoted the degradation of InsR through lysosomal degradation pathway (as shown in Fig. 2D).

### 3.5 Overexpression of EphB4 in C57 mice fed normal diet interferes with glucose tolerance and insulin tolerance

EphB4 was overexpressed in mice fed normal diet by tail vein injection of adenovirus. Without affecting the body weight of mice, the dose of adenovirus injection was 2.5E+7 PFU (PFU: plaque forming unit). One week after injection, glucose tolerance and insulin tolerance were analyzed, and then tissues were harvested. The mice were fasted for 6 hours before tissue harvesting, and some of the mice were injected with insulin (0.5 U/kg) first, and then sacrificed after 5min. The level of phosphorylated Akt was detected by Western blotting.

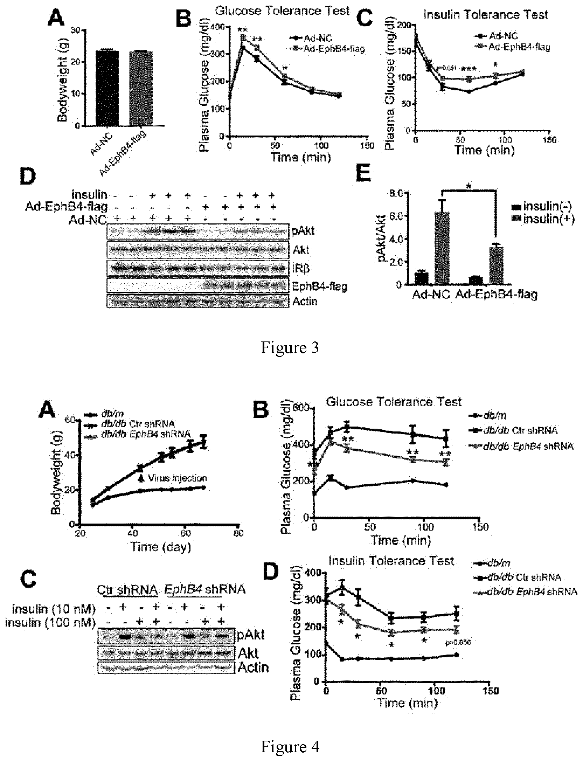

The results showed that overexpression of EphB4 decreased glucose tolerance and clearance ability of mice (as shown in Fig. A, B and C of Fig. 3).

The results of Western blotting showed that overexpression of EphB4 decreased insulin sensitivity of mouse liver tissue (as shown in Fig. D and E of Fig. 3, where E was the result of gray statistical analysis of D, and the calculated value was the gray ratio of phosphorylated Akt to corresponding Akt, ^{∗} p < 0.05).
3.6 Knocking down the expression of *EphB4* can improve glucose tolerance and insulin tolerance in db/db mice
shRNA targeting *EphB4* gene was injected into diabetic *db*/*db* mice via tail vein with lentivirus as a vector, and the expression of *EphB4* was also knocked down in *db*/*db* mice without affecting the body weight. Each mouse was injected with adenovirus at a dose of 2E+7 PFU. Two weeks after injection, glucose tolerance and insulin tolerance were analyzed.

The results showed that knocking down the expression of *EphB4* in *db*/*db* mice could significantly improve glucose tolerance and clearance ability of mice (as shown in Fig. Band D of Fig. 4).
3.7 Knocking down the expression of *EphB4* can improve insulin resistance induced by Hyperinsulinemia

The primary hepatocytes of mice with low *EphB4* expression were isolated and treated with high concentration of insulin (100 nM) for 6 hours to make the hepatocytes in a state of insulin resistance, and then was treated with low concentration of insulin (10 nM) for 20 min. Then the cells were collected and the level of phosphorylated Akt was detected.

The results showed that knocking down the expression of *EphB4* could improve insulin resistance of hepatocytes induced by high concentration of insulin (as shown in Fig. 4C).
3.8 Liver specific knockout of *EphB4* can improve glucose tolerance and clearance ability in mice.

Through the technology platform of Biocytogen, the transgenic mice with flanking insertion of LoxP site into exon 1 of *EphB4* gene for tissue specific knockout were constructed by CRISPR-Cas9 technology. The specific operation procedure is as follows: the target sequence of mouse *EphB4* in genome was amplified and sequenced, and the CRISPR/Cas9 vector plasmid aiming at the target sequence was designed and constructed, and the activity was detected by the company's self-made kit. The targeting vector of conditional knockout of *EphB4* gene was designed and constructed by selecting highly active sgRNA/Cas9 target sites sequence information (see "experimental materials" for details). sgRNA/Cas9 mRNA and targeting vector were injected into the pronucleus of mouse fertilized eggs, and the fertilized eggs were transplanted into the fallopian tubes of surrogate mice. The genotypes of F0 mice with conditional knockout of *EphB4* gene were identified after birth, and the F1 mice with conditional knockout of *EphB4* gene were obtained. The F 1 mice were verified by PCR, southern blotting hybridization, and sequencing to identify genotypes. The F1 mice were mated with the existing liver tissue-specific expression of Cre recombinase mice (Alb-Cre mice), and then the offspring were self-mated, and finally the liver tissue-specific knockout *EphB4* mice were obtained. The liver and muscle tissues of mice were detected by real-time quantitative PCR and Western blotting, and the mice were fed with high fat diet for glucose tolerance tests and insulin tolerance tests.

Similarly, after fasting for 6 hours, some mice were injected with insulin and sacrificed after 5min, and the level of phosphorylated Akt in liver tissue of mice was detected.

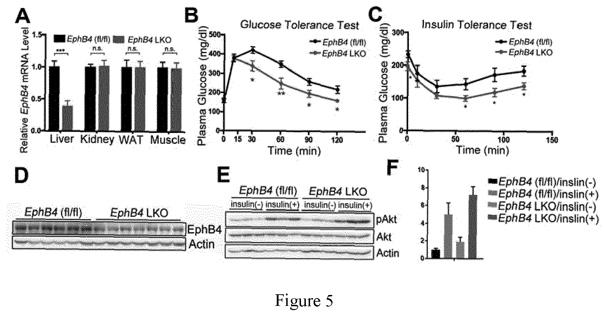

Results: Real-time quantitative PCR detection showed that the mRNA level of *EphB4* in liver tissue decreased significantly, while there was no significant change in kidney, white adipose tissue and muscle tissue (as shown in Fig. A of Fig. 5), and the level of EphB4 protein was also significantly decreased.

Under the condition of feeding high fat diet, *EphB4* LKO mice had better glucose tolerance and clearance ability than that of control mice (as shown in Fig. B and C of Fig. 5). Moreover, there was a higher level of phosphorylated Akt in the liver tissue of knockout mice (as shown in Fig. E of Fig. 5). Fig. 5F is the result of grayscale analysis of Fig. 5E, and the calculated value is the ratio of phosphorylated Akt to corresponding Akt gray level.

The above embodiments only describe the preferred mode of the invention and do not limit the scope of the invention. Without departing from the design spirit of the invention, all kinds of deformations and improvements made by ordinary technicians in the field to the technical scheme of the invention shall fall within the scope of protection determined in the claims of the invention.

## Claims

1. Use of erythropoietin-producing hepatocyte receptor B4 in screening or preparation of a biological agent or a drug for increasing insulin sensitivity, wherein the erythropoietin-producing hepatocyte receptor B4 is used as a target.

2. The use according to claim 1, **characterized in that** said biological agent or drug is used for preventing, alleviating or treating insulin resistance or a disease related to insulin resistance.

3. The use according to claim 2, **characterized in that** said insulin resistance or disease related to insulin resistance is diabetes mellitus, hyperinsulinemia, lipid metabolism disorder, obesity or glucose intolerance.

4. The use according to claim 1, **characterized in that** said biological agent or drug is used for inhibiting the interaction of EphB4 with insulin receptors, or increasing protein level of insulin receptors or level of phosphorylated Akt, or enhancing ability of glucose tolerance and clearance.

5. Use of erythropoietin-producing hepatocyte receptor B4 in the preparation of insulin-sensitized mouse model.

6. The use according to claim 5, **characterized in that** CRISPR-Cas9 technology is used to construct a transgenic mice in which *EphB4* gene was knocked out tissue-specifically and two flanks of first exon were inserted with LoxP site, thereby an insulin-sensitized mouse model with *EphB4* tissue-specifically knockout is obtained.

7. The use according to claim 6, **characterized in that** said insulin-sensitized mouse model with *EphB4* tissue-specifically knockout has enhanced ability of glucose tolerance and clearance.

8. The use according to claim 7, **characterized in that** said tissue is liver.
